# EUROPEAN PATENT SPECIFICATION

(11) **EP 3 056 517 B1**
(45) Date of publication and mention of the grant of the patent: **29.11.2017**
(21) Application number: 16162318.6
(22) Date of filing: 03.09.2009
(51) Int. Cl.: C07K 16/42, C12N 5/10, C12P 21/08, G01N 33/53

(54) **MONOCLONAL ANTIBODY AND IMMUNOASSAY USING THE SAME**
MONOKLONALER ANTIKÖRPER UND IMMUNASSAY DAMIT
ANTICORPS MONOCLONAL ET IMMUNODOSAGE L'UTILISANT

(30) Priority: 05.09.2008 JP 2008228370; 23.10.2008 JP 2008272642; 07.11.2008 JP 2008286521
(43) Date of publication of application: 17.08.2016
(62) Divisional of application: 09811288.1
(73) Proprietor: Sekisui Medical Co., Ltd., Tokyo 103-0027 (JP)
(72) Inventor: Takahashi, Yuki, Ibaraki, 301-0852 (JP); Shimizu, Tomo, Ibaraki, 301-0852 (JP); Takahashi, Hiroshi, Ibaraki, 301-0852 (JP); Nakamura, Yasushi, Ibaraki, 301-0852 (JP)
(74) Representative: Hoffmann Eitle

(56) References cited:
- EP-A2- 0 161 638
- EP-A2- 0 304 329
- WO-A1-93/16729
- AbD Serotec: "Datasheet: MCA1894", , 31 December 2007 (2007-12-31), pages 1-3, XP002663326, Retrieved from the Internet: URL:http://www.abdserotec.com/catalog/data sheetPdf.ashx?ProductID=175741&EntityID=0 [retrieved on 2011-11-11]
- MARUYAMA ET AL: "Activation of human B cells and inhibition of their terminal differentiation by monoclonal anti-mu antibodies.", THE JOURNAL OF IMMUNOLOGY, vol. 135, no. 1, 1 July 1985 (1985-07-01), pages 192-199, XP055011749, ISSN: 0022-1767
- POULETTY PH ET AL: "An anti-human mu chain monoclonal antibody: Use for detection of IgM antibodies to toxoplasma gondii by reverse immunosorbent assay", JOURNAL OF IMMUNOLOGICAL METHODS, ELSEVIER SCIENCE PUBLISHERS B.V.,AMSTERDAM, NL, vol. 76, no. 2, 11 February 1985 (1985-02-11), pages 289-298, XP023992584, ISSN: 0022-1759, DOI: 10.1016/0022-1759(85)90306-0 [retrieved on 1985-02-11]

## Description

### Field of the Invention

The present invention relates to the use of a monoclonal antibody that is capable of reacting specifically with human immunoglobulin M (IgM) and inducing agglutination based on an antigen-antibody reaction with human immunoglobulin M in solution, and a functional fragment derived from the said monoclonal antibody, as well as an immunoassay using the said monoclonal antibody or functional fragment. The present disclosure further relates to a hybridoma producing the said monoclonal antibody.

Furthermore, the present invention relates to the use of an agent for suppressing non-specific reactions and an immunoassay using the same, and more specifically, an agent for suppressing non-specific reactions caused by human IgM and an immunoassay using the same.

### Background of the Invention

An example of widely used assay methods in the diagnostics field is the immunoassay of detecting an analyte (a substance to be tested for) that is present in a test sample by using an antibody raised against the analyte in advance. In particular, the immunoagglutination assay is a method of qualitatively or quantitatively measuring an analyte in a sample based on the degree of agglutination of the lattice-like immunocomplex formed through the bridging of the analytes by the antibody. Since immunoagglutination assay is suited for optical detection and can easily be automated, it is widely applied to test a variety of clinical test items.

Human IgM, produced in the earliest stages of immune response, is present in plasma at about 50 to 200 mg/dL (500 to 2000 µg/mL), and represents a disease marker that could potentially indicate a variety of diseases such as multiple myeloma and protein-losing enteropathy when its level is lower than the normal level, or collagen disease and macroglobulinemia when its level is higher than the normal level.

A number of diagnostic products that are based on the principle of the immunoagglutination assay and intended for human IgM also have been put to practical use, but the antibodies used in such products have been polyclonal so far.

Polyclonal antibody is a collection of antibodies having diverse reactivities, and binds to multiple epitopes on an analyte molecule. Therefore polyclonal antibody has a strongly agglutinating property and is best suited for immunoagglutination assay of an analyte that does not have multiple copies of a same epitope within the molecule. However, polyclonal antibody that binds to multiple epitopes suffers from low specificity. This is actually illustrated in the example of human IgM. Since there are five structurally similar immunoglobulins (including immunoglobulin G) in the body, preparation of IgM-specific polyclonal antibody would require a procedure for removing antibody fractions that cross-react with other immunoglobulins, which is extremely cumbersome and laborious.

On the other hand, monoclonal antibody, which is comprised of a single antibody and has high specificity as it binds to a specific epitope, is easily prepared and performs consistently. Moreover, since modification or alteration of monoclonal antibodies by means of genetic engineering has become easier in recent years, monoclonal antibodies are becoming increasingly important not only for diagnosis but also in the field of therapeutics, and their application areas are expanding further.

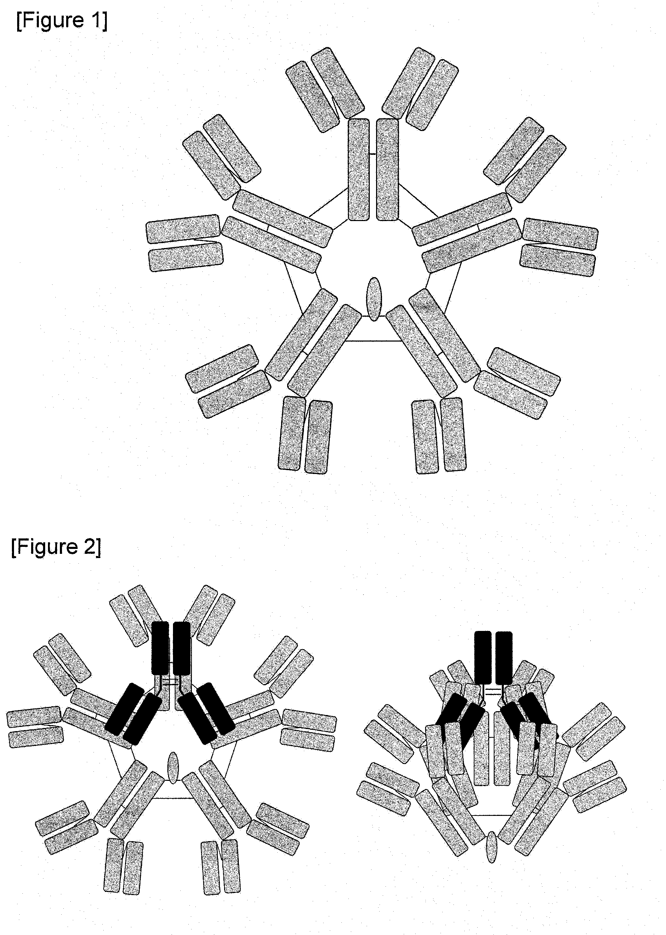

In principle, a monoclonal antibody may be used in an immunoagglutination assay for an analyte having multiple copies of a same epitope. However, it has been believed that an anti-IgM monoclonal antibody cannot induce agglutination efficiently with immunoglobulin M which has ten copies of a same epitope within its molecule, and monoclonal antibodies have not been so far used in the immunoagglutination assay methods. As shown in Figure 1, the plasma IgM has a star-shaped configuration in which five of the Y-shaped unit structure are arranged with their tails in the center and the heads on the periphery. The Y-shape is the unit structure of immunoglobulin, and it is well known that its hinge region is flexible. Furthermore, as shown in the electron microscopic image of Non-Patent Document 1, the five Y-shaped unit structures can assume (b) the flat star-shaped conformation with the tails of the Y-shape in the center and the heads on the periphery or (c) the conformation described as "crab-shaped" in which the heads of the Y-shape are pointing towards the same direction from the base that anchors the tails, which confirms that the region that anchors the tail of the Y-shape is also flexible.

Since the region that anchors each of the subunits within an IgM molecule is flexible and the three-dimensional structure of the molecule changes easily as explained above, an anti-IgM monoclonal antibody would not form inter-molecular bridge by binding two IgM molecules, but instead mostly form so-called intra-molecular bridge by binding the epitope at two locations on a single IgM molecule as shown in Figure 2. It has been therefore believed that an anti-IgM monoclonal antibody would not be able to efficiently induce immunoagglutination with human IgM, and polyclonal antibodies that bind to multiple epitopes have been reluctantly used. Even though there have been examples where monoclonal antibodies are used, they only represent cases in which multiple monoclonal antibodies are used in combination in order to produce the effect of a polyclonal antibody (e.g. Patent Document 4, Example 3). Thus, a monoclonal antibody having a property of agglutinating human IgM on its own has not been known. Moreover, the screening processes in the production of hybridomas that produce anti-human IgM monoclonal antibodies have been previously carried out based on the strengths of reactivity assessed under the condition where either the human IgM or the anti-human IgM monoclonal antibody is immobilized on a solid phase; i.e. the reactivity between them in solution has not been used as an index (Patent Documents 1 - 3).

Advances in the technologies in the recent years have made it possible to produce antibodies against diverse substances, and thus the number of usages of diagnostic reagents based on the immunoassays is continuing to increase. Accordingly, the levels of performance required in the reagents are becoming higher than ever, and there are newly arising needs for strictly preventing non-specific reactions.

A non-specific reaction refers to a phenomenon in which factor(s) contained in the test sample facilitate bonding that is not based on a specific reaction, or inhibit specific immunoreactions, which could cause erroneous assay results. Factors that are known to cause non-specific reactions include the heterophile antibodies and the rheumatoid factors (RF).

Heterophile antibody is a collective term for the human antibodies showing reactivity against the animal-derived antibodies which are the main components of immunoassays. Atypical example of heterophile antibody is HAMA (human anti-mouse-immunoglobulin antibody). A heterophile antibody could be produced as a result of unknowingly induced sensitization to the antigen, such as through eating, through a contact with the animal, and through an administration of biopharmaceuticals. However, an unsensitized antibody could also possibly exhibit the property of heterophile antibody, and thus much remains to be understood regarding the origins of heterophile antibodies. The rheumatoid factors , on the other hand, appear in rheumatoid arthritis patients and their binding site has also been determined. Therefore, rheumatoid factor is understood as a separate concept from heterophile antibody, but they share the same property of exhibiting reactivity against the animal-derived antibodies, and their identities are both known to be human IgG or IgM as described in Non-Patent Document 2.

As for measures against non-specific reactions caused by hetrophile antibodies, heterophilic blocking reagent HBR comprising an anti-human IgM monoclonal antibody described in Non-Patent Documents 3 and 4 previously became commercially available, and has been used in a variety of clinical tests that employ the principles of immunoassays.

Other previously conceived measures for suppressing non-specific reactions include that of Patent Document 5 which involves an addition of a polyclonal antibody against human IgM natural antibodies, which is prepared in the same animal species as the antibody used in the assay, and that of Patent Document 6 which involves an addition of an antibody against the binding site of the rheumatoid factor, which is derived from any of the various animals. However, use of a polyclonal antibody requires a large amount of antigen over a long period of time to immunize the animal. Moreover, the former can only suppress non-specific reactions caused by the natural antibodies, and the latter has only a limited effect of suppressing non-specific reactions because the binding site of the rheumatoid factor recognized therein has a variable nature in the first place. In fact, as the numbers of tests and test items that employ immunoassay increase, it is becoming evident that the conventional measures for suppressing non-specific reactions do not work effectively for some samples.

EP 0304329 describes anti human IgM monoclonal antibody 10C9. WO 9316729 describes monoclonal anti human IgM antibody for reducing xenoreactive IgM natural antibodies.

Maryuama et al., 1985, The Journal of Immunology, vol. 135, no. 1, p. 192-199, describes 3 murine anti human IgM monoclonal antibodies, DA4.4, AB.4 and 145.8.

Pouletty et al. 1985, Journal of Immunological Methods, vo. 76, no. 2, p. 289-298, describes anti human IgM monoclonal antibody Tibi 82 McAb.

### Prior Art Documents

### Patent Documents

Patent Document 1: JP Patent Application Publication S60-42400
Patent Document 2: JP Patent Application Publication S63-58260
Patent Document 3: JP Patent Application Publication H06-504424
Patent Document 4: JP Patent Application Publication S60-237363
Patent Document 5: JP Patent 4065600
Patent Document 6: JP Patent Application Publication H07-012818

### Non-Patent Documents

Non-Patent Document 1: Roitt, Essential Immunology, third edition in the original language, Figure 2.16.
Non-Patent Document 2: Rinsho Kagaku, Volume 23 Supplement 175a-1 - 175a-10 (1994).
Non-Patent Document 3: Advertisement material for the heterophilic blocking reagent HBR (Nagase & Co. Ltd., 1993)
Non-Patent Document 4: CLIN. CHEM. 45/7, 942 - 956 (1999)

### Disclosure of the Invention

### Problems to Be Solved by the Invention

An objective of the present invention is to provide a use of an anti-human IgM monoclonal antibody that reacts specifically with human IgM and is capable of inducing immunoagglutination based on an antigen-antibody reaction in solution with human IgM, functional fragments of the said monoclonal antibody, and immunoassays using the said monoclonal antibody or fragments.

A further objective of the present invention is to provide a use of an agent for suppressing non-specific reactions caused by human IgM, and immunoassays using the said agent.

### Means of Solving the Problem

The present inventors have conducted extensive research to attain the above objectives, and by using the reactivity in solution as an evaluation index in the screening for monoclonal antibodies that react with human IgM, the inventors have been able to find a novel monoclonal antibody that is capable, by itself (i.e. by being a sole monoclonal antibody), of providing bridging between human IgM molecules to cause agglutination. This has led to the completion of the present invention. The monoclonal antibody thus obtained did not necessarily exhibit high reactivity in the conventional evaluation procedure in which the antigen was immobilized on a solid phase.

The present inventors have also made a surprising finding that non-specific reactions, even those that cannot be blocked by the conventional measures, may be strongly suppressed by the said anti-human IgM monoclonal antibody which is capable of agglutinating human IgM by itself. Thus, another objective of the present invention is to provide a use of an agent for suppressing non-specific reactions that comprises the anti-human IgM monoclonal antibody capable of agglutinating human IgM by itself.

A further objective of the present invention relates to immunoassays, characterized in that suppression of non-specific reactions and accuracy of measurements in the immunoassays that are based on antigen-antibody reactions are achieved by the addition of the anti-human IgM monoclonal antibody capable of agglutinating human IgM by itself.

Specifically, the present invention comprises the following.
(1) An immunoassay for determining the quantity of an analyte in a sample based on an antigen-antibody reaction between the analyte and an assay antibody or an assay antigen,
   wherein non-specific reactions are suppressed by the addition of an anti-human IgM monoclonal antibody that reacts specifically with human IgM and is capable of inducing immunoagglutination by itself based on an antigen-antibody reaction with human IgM in solution, or a functional fragment derived from said anti-human IgM antibody,
   wherein the functional fragment comprises a Fab region derived from the said anti-human IgM antibody,
   and wherein the analyte is not human IgM.
(2) The immunoassay according to (1), wherein the anti-human IgM monoclonal antibody is selected from (a) or (b) below:
   (a) A monoclonal antibody produced by FERM BP-11134 hybridoma;
   (b) An antibody that shows a cross-reactivity with the antibody according to (a) above.
(3) The immunoassay according to any of (1) to (2), wherein the assay antibody or the assay antigen is supported on an insoluble carrier.
(4) The immunoassay according to (3), wherein the insoluble carrier comprises latex, colloidal metal, or silica.
(5) The immunoassay according to any of (1) to (4), wherein the anti-human IgM monoclonal antibody is put into contact with the sample before a step of bringing the assay antibody or the antigen into contact with the sample.
(6) Use of an anti-human IgM monoclonal antibody that reacts specifically with human IgM and is capable of inducing immunoagglutination by itself based on an antigen-antibody reaction with human IgM in solution, or a functional fragment derived from said anti-human IgM antibody for suppressing non-specific reactions in an immunoassay for determining the quantity of an analyte in a sample based on an antigen-antibody reaction between the analyte and an assay antibody or an assay antigen,
   wherein the functional fragment comprises a Fab region derived from the said anti-human IgM antibody,
   and wherein the analyte is not human IgM.
(7) The use according to (6), wherein the anti-human IgM monoclonal antibody is put into contact with the sample before a step of bringing the assay antibody or the antigen into contact with the sample.

Also disclosed herein is:
(1) An anti-human IgM monoclonal antibody that reacts specifically with human IgM and is capable of forming an immune aggregate based on an antigen-antibody reaction with human IgM in solution.
(2) The anti-human IgM monoclonal antibody according to (1) above obtained by a method of producing an anti-human IgM monoclonal antibody comprising the following steps:
   Step 1) A step of obtaining hybridomas that produce anti-human IgM antibodies, by using human IgM as an antigen.
   Step 2) A step of bringing the antibodies produced by the hybridomas obtained in Step 1 into contact with human IgM in solution, and selecting a hybridoma that produces an antibody capable of forming an immune aggregate based on an antigen-antibody reaction with human IgM in the said solution.
   Step 3) A step of obtaining the antibody produced by the hybridoma selected in Step 2.
(3) An antibody selected from (a) or (b) below:
   (a) A monoclonal antibody produced by FERM BP-11134 hybridoma.
   (b) The antibody according to (1) that shows a cross-reactivity with the antibody according to (a) above.
(4) A functional fragment derived from the anti-human IgM monoclonal antibody according to any of (1) to (3) above.
(5) An immunoagglutination assay for determining quantity of human IgM in a sample, comprising a use of the monoclonal antibody according to any of (1) to (3) above and/or the functional fragment derived from the monoclonal antibody according to (4) above.
(6) An immunoagglutination assay reagent and immunoagglutination assay kit for determining quantity of human IgM in a sample, comprising the monoclonal antibody according to any of (1) to (3) above and/or the functional fragment derived from the monoclonal antibody according to (4) above.
(7) A hybridoma that produces the monoclonal antibody according to (1) or (2) above.
(8) FERM BP-11134 hybridoma.
(9) A method of producing an anti-human IgM monoclonal antibody comprising the following steps:
   Step 1) A step of obtaining hybridomas that produce anti-human IgM antibodies, by using human IgM as an antigen.
   Step 2) A step of bringing the antibodies produced by the hybridomas obtained in Step 1 into contact with human IgM in solution and selecting a hybridoma that produces an antibody capable of forming an immune aggregate based on an antigen-antibody reaction with human IgM in the said solution.
   Step 3) A step of obtaining the antibody produced by the hybridoma selected in Step 2.
(10) An agent for suppressing non-specific reactions caused by human IgM, comprising the monoclonal antibody according to any of (1) to (3) above and/or the functional fragment derived from the monoclonal antibody according to (4) above.
(11) An immunoassay for determining quantity of an analyte in a sample based on an antigen-antibody reaction between the analyte and an assay antibody or an assay antigen, wherein non-specific reactions caused by human IgM that do not include the said antigen-antibody reaction are suppressed by an addition of an agent for suppressing non-specific reactions caused by human IgM, the said agent comprising the monoclonal antibody according to any of (1) to (3) above and/or the functional fragment derived from the monoclonal antibody according to (4) above.
(12) The immunoassay according to (11) above, wherein the assay antibody or assay antigen is supported on an insoluble carrier.
(13) The immunoassay according to (12) above, wherein the insoluble carrier comprises latex, colloidal metal, or silica.
(14) An immunoassay reagent and an immunoassay kit, comprising:
   an agent for suppressing non-specific reactions caused by human IgM which comprises the monoclonal antibody according to any of (1) to (3) above and/or the functional fragment derived from the monoclonal antibody according to (4) above; and
   an assay antibody or an assay antigen for determining quantity of an analyte in a sample.
(15) The immunoassay reagent and the immunoassay kit according to (14) above, wherein the assay antibody or assay antigen is supported on an insoluble carrier.
(16) The immunoassay reagent and the immunoassay kit according to (15) above, wherein the insoluble carrier comprises latex, colloidal mental, or silica.

### Effects of the Invention

The monoclonal antibody used in the present invention has made it possible to provide a method for specifically detecting human IgM, as well as a high-quality and inexpensive reagent and kit for assaying immunoagglutination of human IgM.

Moreover, since the present disclosure's method of screening for the monoclonal antibody evaluates the degree of agglutination in solution as an index for selecting the antibody, the method is efficient and able to select a monoclonal antibody of interest at a high precision.

Moreover, since the present disclosure's agent for suppressing non-specific reactions does not form a 1:1 complex with IgM that represents intra-molecular bridging, the agent can neutralize the interference of IgM efficiently. Thus, the immunoassay using the agent used in the present invention can suppress not only the ordinary types of non-specific reactions caused by human IgM but also those that could not be prevented by the conventional measures. Therefore, the present invention enables more accurate measurements for a wide range of test items and test samples than the conventional measurement methods, and may be used suitably for the immunoassay which are employed in an increasing number of clinical tests.

### Brief Descriptions of the Figures

[Figure 1] A structural model of a human IgM.
[Figure 2] The binding modes of a human IgM and a conventional anti-human IgM monoclonal antibody.
[Figure 3] Results of the immunoagglutination measurement tests using the anti-human IgM monoclonal antibody of the present invention (Example 1).
[Figure 4] Effects of the present disclosure's agent for suppressing non-specific reactions and the control agents for blocking non-specific reactions, on the assay of a normal specimen with the latex reagent for PSA testing.
[Figure 5] Blocking effects of the present disclosure's agent and the control agents for blocking non-specific reactions on the assay of a non-specific reaction specimen with the latex reagent for PSA testing.
[Figure 6] Blocking effects of the present disclosure's agent and the control agents for blocking non-specific reactions on the measurement of a non-specific reaction specimen with the reagent for insulin testing.

### Best Mode for Carrying Out the Invention

### Anti-human IgM monoclonal antibody

The anti-human IgM monoclonal antibody used in the present invention is not bound by particular limitations as long as it has the property of reacting specifically with human IgM, forming an inter-molecular bridge between human IgM molecules in solution, and being capable of performing an immunoagglutination assay as a sole monoclonal antibody. "Reacting specifically with human IgM" as used herein means that the monoclonal antibody is subject to an antigen-antibody reaction with human IgM but not with the other human immunoglobulins. A search for a monoclonal antibody against IgM has not been previously carried out from the viewpoint of whether it can cause immunoagglutination, and therefore the anti-human IgM monoclonal antibody used in the present invention that can agglutinate human IgM by itself is novel.

Moreover, the conventional anti-IgM monoclonal antibodies did not provide bridging between two immunoglobulin molecules and were only capable of forming an intra-molecular bridge within a single immunoglobulin molecule. However, the monoclonal antibody used in the present invention that can by itself agglutinate human IgM can form an inter-molecular bridge between human IgM molecules, and it is therefore capable of strongly suppressing non-specific reactions caused by human IgM, and as described below, it may be used suitably as an agent for suppressing non-specific reactions.

Specific examples of such anti-human IgM monoclonal antibodies used in the present invention include the monoclonal antibody produced by the FERM BP-11134 hybridoma. Furthermore, antibodies showing a cross-reactivity with the monoclonal antibody produced by the FERM BP-11134 hybridoma are also comprised in the scope of the antibody used in the present invention. The antibodies showing a cross-reactivity with the monoclonal antibody produced by the FERM BP-11134 hybridoma include those that may specifically recognize the amino acid sequence of the same epitope (antigenic determinant) as the said monoclonal antibody.

### Functional fragment

In the present invention, any functional fragment comprising a Fab region derived from the said monoclonal antibody may be used, whether the Fab region is obtained by an enzymatic digestion of the said monoclonal antibody, by genetic engineering, or by other means. Thus, in the functional fragment used in the present invention, the term "functional" specifically means that the fragment has the capacity to bind to human IgM.

### Test sample

The test sample for the immunoagglutination assay for human IgM using the anti-human IgM monoclonal antibody or the functional fragment thereof used in the present invention may be any biological sample, such as a body fluid, e.g. blood, serum, plasma and urine.

### Assay method

The immunoagglutination assay for human IgM according to the present disclosure is not bound by any particular limitations, as long as it tests for human IgM by mixing in some manner the anti-human IgM monoclonal antibody used in the present invention and the test sample and thus inducing immunoagglutination. In order to further facilitate the immunoagglutination, another anti-human IgM monoclonal antibody that recognizes a different epitope from that of the said monoclonal antibody may also be used together, and there is no particular limitation as to the property of this additional monoclonal antibody.

### Immunoagglutination assay reagent and assay kit

The reagent and kit for immunoagglutination assays for human IgM that used in use the anti-human IgM monoclonal antibody or the functional fragment thereof of the present invention may contain a component for buffering the ionic strength, osmotic strength or the like of the sample, or a component for enhancing the immunological agglutination. Examples of the component for buffering the ionic strength, osmotic strength or the like of the sample include acetic acid, citric acid, phosphoric acid, Tris, glycine, boric acid, carbonic acid, and Good's buffer, as well as sodium salt, potassium salt, and calcium salt. Examples of the component for enhancing the immunological agglutination include polyethylene glycol, polyvinylpyrrolidone, and phospholipid polymer.

The reagent and kit for immunoagglutination assays for human IgM that use the anti-human IgM monoclonal antibody or the functional fragment thereof used in the present invention are compatible with optical measurements, performed on a spectrophotometer, an automatic analyzer of the Hitachi corporation that uses the principle of spectrophotometry, a general automatic analyzer such as the TBA series of the Toshiba corporation, the BM series of the JEOL corporation, the AU series of the Olympus corporation, and CP2000 of the Sekisui Medical corporation, a device for analyzing the near-infrared wavelength range (e.g. LPIA of the Mitsubishi Kagaku Iatron corporation), a device for measuring the intensity of scattered light (e.g. the BN System of the Dade Behring corporation), or the like.

There is no particular limitation as to the technologies used for the preparation of the anti-human IgM monoclonal antibody used in the present invention, but generally they involve production of hybridomas. In the evaluation process for selecting the anti-human IgM monoclonal antibody used in the present invention, the antibodies are screened based on the degrees of agglutination of human IgM they could induce in solution. Consequently, the anti-human IgM monoclonal antibody thus obtained will have the property of reacting specifically with human IgM and agglutinating human IgM by itself.

Any structural alteration or modification may be allowed to the prepared anti-human IgM monoclonal antibody used in the present invention, as long as the alteration or modification does not greatly impair the monoclonal antibody's characteristics with respect to the reactivity with human IgM.

### Hybridoma

The hybridoma used for producing the anti-human IgM monoclonal antibody of the present disclosure may be any hybridoma that is capable of producing an anti-human IgM monoclonal antibody that can react specifically with human IgM and induce immunoagglutination with human IgM based on an antigen-antibody reaction in solution, and the said hybridoma may be selected on the basis that the monoclonal antibody produced by it is highly capable of inducing immunoagglutination with human IgM in solution. Thus, the anti-human IgM monoclonal antibody used in the present invention may be produced via the following Steps 1) to 3).
Step 1) A step of establishing hybridomas producing anti-human IgM antibodies by using human IgM as an antigen.
Step 2) A step of bringing the antibodies produced by the hybridomas obtained in Step 1 into contact with human IgM in solution and selecting a hybridoma producing an antibody capable of forming an immune aggregate based on an antigen-antibody reaction with human IgM in the said solution.
Step 3) A step of obtaining the antibody produced by the hybridoma selected in Step 2.

Examples of the hybridomas selected in Step 2 include FERM BP-11134.

The screening of the hybridomas in the above production process may, more specifically, be carried out by allowing the purified antibodies to react with human IgM in solution and selecting the hybridomas based on the corresponding antibodies' differences in the ability to induce immunoagglutination in solution. To select a hybridoma producing the antibody used in the present invention more efficiently, pre-selection of the hybridomas producing anti-human IgM antibodies may be performed, prior to the selection process mentioned above, by using, for example, the culture supernatants of the hybridomas instead of the purified antibodies on human IgM on a solid phase, as in the ELISA etc.

The antibody obtained through the above-described selection process will have the property of specifically reacting with human IgM and being capable on its own of forming an inter-molecular bridge between human IgM molecules in solution and hence forming an immune aggregate.

### Applications

The anti-human IgM monoclonal antibody used in the present invention may be applied for any purposes that make use of its property of reacting specifically with human IgM and agglutinating human IgM by itself. Preferable examples include a use in the immunoagglutination assay for human IgM mentioned above as well as a use as an agent for suppressing non-specific reactions caused by human IgM. The anti-human IgM monoclonal antibody used in the present invention may be suitably used as an agent for suppressing non-specific reactions caused by human IgM, because it does not cause 1:1 intra-molecular bridging with a human IgM molecule, and therefore it can efficiently neutralize human IgM. It is not clear by what mechanism the antibody used in the present invention is able to suppress even those non-specific reactions caused by IgM that could not be suppressed by the conventional agents for inhibiting non-specific reactions, but it is believed that its ability to agglutinate human IgM in solution is highly relevant to the mechanism. For example, it is possible that the anti-human IgM monoclonal antibody that could agglutinate human IgM in solution recognizes an epitope that exists universally among human IgM molecules, regardless of whether they are of the heterophile antibody type, of the rheumatoid factor (RF) type, or other.

Thus, the non-specific reactions caused by human IgM, in the context of the present invention, refer to all non-specific reactions caused by human IgM, including those whose mechanisms of action have been known such as human IgM in the form of a heterophile antibody or a rheumatoid factor (RF) reacting with an animal-derived antibody, as well as those whose mechanisms of action remain unclear. Thus, the present disclosure's agent for suppressing non-specific reactions is intended for all non-specific reactions in which a causative factor is human IgM.

The use of the anti-human IgM monoclonal antibody used in the present invention as an agent for suppressing non-specific reactions can be effective in solution as well as on solid phase. A use of the present disclosure's agent for suppressing non-specific reactions in solution may be, for example, a case where the said agent is added to a sample solution and allowed to react with human IgM in the solution to cause agglutination of the human IgM. A use of the said agent on solid phase may be, for example, a case where a solution containing the said agent is added to a sample pad for immuno-chromatography and dried and immobilized thereon in advance, and human IgM contained in a sample solution which is applied to and passes through the said sample pad will be trapped by the said agent that has been immobilized.

The present disclosure's agent for suppressing non-specific reactions can be used in an immunoassay for determining the quantity of an analyte in a sample based on an antigen-antibody reaction between the analyte and an assay antibody or an assay antigen, and through this usage, non-specific reactions caused by human IgM, which do not include the said antigen-antibody reaction, may be suppressed effectively, and accurate immunological measurements may be thus achieved.

The test sample for the immunoassay of the present invention, which is characterized by the addition of the anti-human IgM monoclonal antibody capable by itself of agglutinating human IgM for suppressing non-specific reactions and for obtaining accurate measurements, may be any of the diverse human biological samples in which non-specific reactions caused by human IgM could possibly occur. Examples of the test sample include body fluids such as blood, serum, plasma and urine. Also, the analyte could be any molecule as long as it could be subjected to an antigen-antibody reaction, and examples of the analyte include CRP (C-reactive protein), Lp (a), MMP3 (matrix metalloproteinase 3), type IV collagen, PSA (prostate-specific antigen), BNP (brain natriuretic peptide), insulin, microalbumin, cystatin C, antiphospholipid antibody, anti-Treponema pallidum antibody, FDP (fibrin/fibrinogen degradation product), D-dimer, SF (soluble fibrin), TAT (thrombin-antithrombin III complex), factor XIII, and pepsinogens I and II, as well as haptens such as the following drugs: phenytoin, phenobarbital, carbamazepine, valproate, and theophylline. However, this invention's principle does not allow human IgM to be included in these examples of the analyte.

The present disclosure's anti-human IgM monoclonal antibody capable by itself of agglutinating human IgM, used as an agent for suppressing non-specific reactions caused by IgM, may be added to a solution or to a solid phase. There is no particular limitation as to the concentration at which the said agent is added as long as it sufficiently produces the effect of suppressing non-specific reactions and does not interfere with the main reaction of the immunoassay, but the agent is preferably used in the concentration range of 0.01 to 10 mg/mL, and more preferably in the concentration range of 0.05 to 1 mg/mL. In an actual assay, non-specific reactions may be more efficiently suppressed if the anti-human IgM monoclonal antibody used in the present invention is put into contact with the test sample before the step of bringing the assay antibody or the assay antigen into contact with the test sample. Other measures for suppressing non-specific reactions or other inhibitor components may also be used in combination.

In the immunoassay of the present invention in which the component that is instrumental in the immunological measurement (assay antibody or assay antigen) is supported on an insoluble carrier, the insoluble carrier may be of any material as long as it is an insoluble material acceptable as a component in a medical test reagent; specific examples include latex particle, colloidal metal, silica, and carbon, and latex particle is especially preferable. The material and the size of the insoluble carrier may be selected as appropriate according to the type of the analyte and the detection principle of the immunoassay of the present invention. In the case of latex particle above, a carrier particle comprising a copolymer made through polymerization of a monomer having a phenyl group and a monomer having a phenyl group and a sulfonic acid salt is preferable, and its average particle size, as measured by a transmission electron microscope, is 0.01 to 1.5 µm, preferably 0.03 to 0.8 µm, and more preferably 0.05 to 0.5 µm.

In the immunoassay of the present invention characterized by the suppression of the effect of non-specific factors on the antibody that binds specifically to the analyte molecule for obtaining accurate measurements, any detection principle may be used, such as enzymatic detection, fluorescent detection, chemiluminescent detection, turbidity-based detection, and the like. Moreover, the manipulations and the evaluations may be performed manually or mechanically. Thus, the immunoassay reagent and assay kit of the present disclosure may be employed in any of the following: ELISA, EIA, chemiluminescent method, immunochromatography, immunoagglutination method, latex enhanced immunoagglutination method, and the like. In particular, the immunoagglutination method and the latex enhanced immunoagglutination method may be performed in the same equipments as those used with the immunoagglutination assay reagent and assay kit for human IgM described above.

Also, the present disclosure's immunoassay reagent and assay kit using the agent for suppressing non-specific reactions caused by human IgM may contain, in addition to the main component, a component for buffering the ionic strength, osmotic strength or the like of the sample, or a component for enhancing the immunoagglutination, which can be the same components as described above for the immunoagglutination assay reagent and assay kit for human IgM.

### Examples

Below, part of the present invention will be explained in detail by giving examples of the method of producing the anti-human IgM monoclonal antibody capable of inducing immunoagglutination of human IgM by itself, the immunoagglutination assay using the said monoclonal antibody, and the immunoassay using the said monoclonal antibody as an agent for suppressing non-specific reactions. However, the present invention is not limited by these examples.

### [Test Example 1] Production of the anti-human IgM monoclonal antibody used in the present invention

### (1) Establishment of the hybridomas that produce anti-human IgM monoclonal antibodies

100 µg of purified human IgM (manufactured by the CHEMICON corporation) was used in a single immunization. In the first immunization, 200 µL of an emulsion prepared by mixing equal volumes of human IgM and Freund's complete adjuvant was injected into the abdominal cavity of each of the BALB/c mice. For an additional immunization, 200 µL of an emulsion similarly prepared by using Freund's incomplete adjuvant was used, and the immunization was repeated three times at intervals of 2 weeks each. The antibody titers of the bloods collected from the retinal veins of the mice were measured by ELISA, and a mouse showing a high titer was selected for cell fusion. Two weeks after the fourth immunization, 100 µg of human IgM dissolved in 200 µL of saline solution was injected into the abdominal cavity of the mouse, and the spleen was removed 3 days later. The spleen was disrupted in RPMI1640 medium, and the spleen cells were then collected through a centrifugation at 1,500 rpm. The cells were washed at least three times in RPMI1640 medium that is free of fetal bovine serum, and then suspended in 2 mL of the RPMI1640 medium containing 15% fetal bovine serum, to produce a spleen cell suspension. After the spleen cells and SP2/O-AG14 myeloma cells were mixed at a ratio of 6 to 1, cell fusion was induced in the presence of 50% polyethylene glycol, and the hybridomas (fused cells) were thus obtained. Following a centrifugation at 1,500 rpm, the pellet was collected and suspended in the GKN solution (2 g of glucose, 0.4 g of potassium chloride, 8 g of sodium chloride, 1.41 g of disodium hydrogen phosphate, and 0.78 g of sodium dihydrogen phosphate dihydrate were dissolved in purified water to make 1 L of GKN solution), washed through centrifugation, and the pellet was collected. The pelleted cells were suspended in 30 mL of RPMI1640 medium containing 15% fetal bovine serum, and 100 µL of this suspension and 200 µl of HAT medium containing the thymus cells of BALB/c mouse (representing feeder cells) at 2.5×10⁶ cells/mL were dispensed to each well of three 96-well microplates. The hybridomas were cultured at 37°C in an incubator with 5% carbon dioxide.

The presence of anti-human IgM antibodies in the culture supernatants was verified by ELISA with human IgM on solid phase. Ten days after the cell fusion, growth of hybridomas was confirmed in every well. In detail, 100 µL of 10 mM phosphate buffer solution (pH 7.2; hereafter abbreviated as PBS) containing 150 mM sodium chloride and 10 µg/mL human IgM was dispensed in each well of the 96-well microplates, and left at 4°C overnight. After these 96-well microplates were washed three times with 300 µL PBS containing 0.05% Tween 20 and 1 % bovine serum albumin, the culture supernatants were added at 50 µL/well, and the plates were left at room temperature for 1 hour. After the plates were washed three times with PBS containing 0.05% Tween 20 (hereafter abbreviated as PBS-T), peroxidase-labelled goat anti-mouse IgG antibody (SouthernBiotech corporation) was added at 50 µL/well, and the plates were left at room temperature for 1 hour. The plates were then washed three times with PBS-T, citric acid buffer solution (pH 5.0) containing 0.2% ortho-phenylenediamine and 0.02% hydrogen peroxide was added thereto at 50 µL/well, the plates were left at room temperature for 15 minutes, and 4.5N sulfuric acid was added at 50 µL/well to stop the reaction. Absorbance in each well at the wavelength of 492 nm was measured, and the wells showing differences from the blank were selected as being positive wells.

The cells were rendered monoclonal by limiting dilution. That is, the hybridomas from the positive wells mentioned above were diluted to 10 cells/mL and 0.1 mL each of these dilutions was placed in a well of 96-well microplates in which BALB/c mouse thymus cells, as feeder cells, had been dispensed at 10⁶ cells/well. HAT medium was used for the first culture and RPMI1640 containing 15% fetal bovine serum was used for the subsequent cultures. Each culture was continued for 10 days at 37°C in an incubator with 5% carbon dioxide. ELISA for selecting positive wells and limiting dilution for establishing monoclonality were each repeated three times, and 26 lines of hybridomas (A to Z) producing anti-human IgM monoclonal antibodies were thus obtained.

### (2) Production of the monoclonal antibodies

Approximately 10⁵ cells from each hybridoma line were transferred to the mouse abdominal cavity that had been pre-treated with pristane, and the ascites generated therein was collected. The collected ascites fluids were centrifuged to remove insoluble materials and mixed with an equal volume of saturated ammonium sulfate solution. The mixtures were stirred overnight, and following a centrifugation, the precipitates were collected. The collected precipitates were dissolved in 20 mM Tris buffer solution (pH 8.0) and dialyzed in the same buffer solution. The dialyzed materials were separately adsorbed to DEAE-Sepharose columns that had been equilibrated with the same buffer solution, and the anti-human IgM monoclonal antibodies were obtained by elution with a 0 to 300 mM concentration gradient of sodium chloride in the same buffer solution. The anti-human IgM monoclonal antibodies produced by the hybridomas A to Z were denoted as the antibodies a to z, respectively, and subjected to the tests described below.

### [Examples of evaluation]

### (1) Evaluation Example 1: Evaluation of reactivity on solid phase (conventional evaluation method)

50 µL of PBS containing human IgM at 1 µg/mL was dispensed in a 96-well microplate and left at room temperature for 1 hour, and the human IgM was thus immobilized on the microplate. The microplate was then subjected to blocking with 200 µL of PBS containing 3% BSA (bovine serum albumin), and following the addition of 50 µL of PBS containing the anti-human IgM monoclonal antibody at 1 µg/mL to the well, it was left at room temperature for 1 hour. The microplate was then washed three times with PBS-T, peroxidase-labelled anti-mouse antibody was added at 50 µL/well, and the microplate was left at room temperature for 1 hour. Following three washes with PBS-T, 50 µL of 2,2'-azino-bis(3-ethylbenzthiazoline-6-sulphonic acid (ABTS) (manufactured by the KPL corporation) substrate solution was added to each well, and the microplate was left at room temperature for 20 minutes. The reaction was stopped by adding 50 µL of 1% SDS solution to each well. Absorbance at the wavelength of 405 nm was measured, and according to the absorbance, the strengths of reactivity of the anti-human IgM monoclonal antibodies were ranked (Table 1).

### (2) Evaluation Example 2: Evaluation of reactivity in solution (evaluation method according to the present disclosure

750 µL each of the anti-human IgM monoclonal antibody solutions (i.e. 100 mM Tris-hydrochloric acid buffer solution, pH 8.0, containing 400 µg/mL purified anti-human IgM monoclonal antibody, 3% polyethylene glycol 6000, 5 mM trisodium citrate and 2.5 mM calcium chloride (anhydride)) was added to 150 µl of PBS containing human IgM at 200 µg/mL, and the mixture was stirred. Ten minutes later, absorbance was measured by using two wavelengths (the main wavelength of 340 nm and the secondary wavelength of 800 nm) in the Hitachi U-3310 spectrophotometer (Absorbance 2). In a control, 750 µL each of the anti-human IgM monoclonal antibody solutions was added to 150 µL of PBS not containing human IgM, the mixture was stirred, and absorbance was measured under the identical condition (Absorbance 1). The change in the absorbance that accompanied the human-IgM-specific immunoagglutination was calculated by subtracting Absorbance 1 from Absorbance 2 (Table 1).

**[Table 1]**

| Antibody | Subtype | Conventional evaluation | | Evaluation according to the present invention | | |
|---|---|---|---|---|---|---|
| | | Absorbance (Abs) | Reactivity rank | Absorbance 1 (mAbs) | Absorbance 2 (mAbs) | Change in absorbance |
| a | G | 0.070 | 23 | 4.1 | 5.3 | 1.2 |
| b | G | 0.069 | 24 | 3.6 | 4.8 | 1.2 |
| c | G | 0.103 | 18 | 4.3 | 4.7 | 0.4 |
| d | G | 0.064 | 25 | 4.0 | 4.8 | 0.8 |
| e | G | 0.416 | 15 | 5.3 | 7.5 | 2.2 |
| f | G | 0.315 | 17 | 6.0 | 12.1 | 6.1 |
| g | G | 0.619 | 8 | 4.6 | 8.3 | 3.7 |
| h | G | 0.509 | 13 | 3.5 | 11.5 | 8.0 |
| i | G | 0.545 | 12 | 5.0 | 10 | 5.0 |
| j | G | 0.710 | 4 | 4.4 | 8.7 | 4.3 |
| k | G | 1.464 | 1 | 5.6 | 10.3 | 4.7 |
| l | G | 0.619 | 8 | 3.9 | 7.6 | 3.7 |
| m | G | 0.084 | 20 | 3.9 | 4.5 | 0.6 |
| n | G | 0.087 | 19 | 3.9 | 5.3 | 1.4 |
| o | G | 0.318 | 16 | 18.3 | 20.6 | 2.3 |
| p | G | 0.080 | 21 | 4.7 | 5.6 | 0.9 |
| q | G | 0.070 | 23 | 4.0 | 4.3 | 0.3 |
| r | G | 0.781 | 3 | 4.6 | 10.9 | 6.3 |
| s | G | 0.603 | 10 | 4.9 | 8.3 | 3.4 |
| t | G | 0.685 | 6 | 5.1 | 161.8 | 156.7 |
| u | G | 0.627 | 7 | 2.9 | 6.5 | 3.6 |
| v | G | 0.078 | 22 | 5.4 | 5.7 | 0.3 |
| w | G | 0.694 | 5 | 5.2 | 8.7 | 3.5 |
| x | G | 0.448 | 14 | 6.7 | 11.4 | 4.7 |
| y | G | 0.567 | 11 | 5.0 | 13.8 | 8.8 |
| z | G | 1.161 | 2 | 14.1 | 16.7 | 2.6 |

### (3) Results of the evaluations

From the change in the absorbance, the antibody t was found to be an anti-human IgM monoclonal antibody that reacts specifically with human IgM and is capable of inducing immunoagglutination based on an antigen-antibody reaction with human IgM in solution. It is noted that, in the conventional evaluation method (i.e. evaluation in a solid-phase condition), the strengths of reactivity of the antibodies k and z were prominent, and although the reactivity of the antibody t was ranked 6th, it was less than half of the reactivity of the antibody k, and therefore it is unlikely that the antibody t used in the present invention would have been selected. At the same time, it can be seen that the capacity to form an immune aggregate based on an antigen-antibody reaction with human IgM in solution does not necessarily coincide with a strong reactivity, and therefore a monoclonal antibody having the property of the present invention would be extremely difficult to identify based only on the conventional evaluation of reactivity in a solid-phase condition. The hybridoma T that produces the antibody t has been deposited under the deposition number of FERM BP-11134.

### [Example 1]

### Verification of immunoagglutination by the anti-human IgM monoclonal antibody and its potential utility as an agent for suppressing non-specific reactions

### (1) Preparation of human IgM solutions

A solution containing human IgM (manufactured by the CHEMICON corporation) at 200 µg/mL was serially diluted in PBS to prepare 100, 50, and 25 µg/mL human IgM solutions.

### (2) Preparation of a solution of the anti-human IgM monoclonal antibody t

100 mM Tris-hydrochloric acid buffer solution (pH 8.0) containing 3% polyethylene glycol 6000, 5 mM trisodium citrate and 2.5 mM calcium chloride (anhydride) (hereafter referred to as the antibody diluting solution) was used to dilute the anti-human immunoglobulin M monoclonal antibody t used in the present invention to a final concentration of 400 µg/mL, to prepare a solution of the anti-human IgM monoclonal antibody t. As a control, a solution of the heterophilic blocking reagent HBR was prepared by diluting the heterophilic blocking reagent HBR (manufactured by the SCANTIBODIES LABORATORY, INC. and imported by the Nagase & Co. Ltd.) which comprises an anti-human IgM monoclonal antibody used as a blocker of non-specific reactions, instead of the anti-human immunoglobulin M monoclonal antibody t, in the antibody diluting solution to a final concentration of 400 µg/mL.

### (3) Assay method

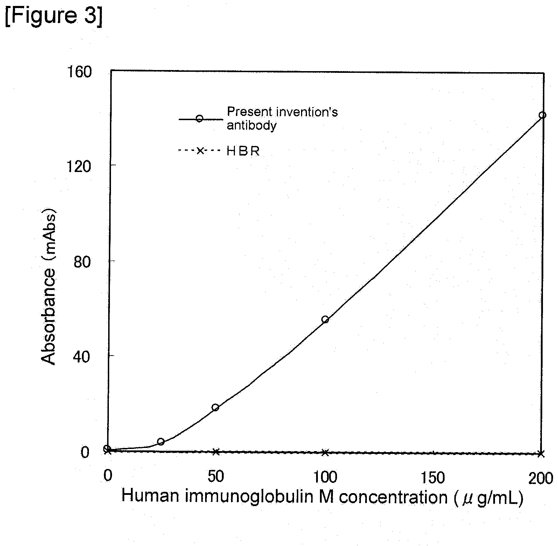

Immunoagglutination assay was performed by using the Hitachi type 7170 automatic analyzer. Specifically, 150 µL of the solution of the anti-human immunoglobulin M monoclonal antibody t prepared in (2) or 150 µL of the solution of the heterophilic blocking reagent HBR was added to 30 µL of the solution of human IgM at each concentration prepared in (1), the mixture was stirred and warmed at 37°C, and the change in the absorbance at the main wavelength of 340 nm and the secondary wavelength of 800 nm over 10 minutes was measured as a measure of reaction strength (mAbs). The results are shown in Figure 3.

### (4) Results

The anti-human IgM monoclonal antibody t used in the present invention (in-house reference number 73224) showed concentration-dependent increase of reaction strength at the human IgM concentration of 25 µg/mL and above, and its ability to carry out a practical immunoagglutination assay for human IgM by itself was thus confirmed. On the other hand, the heterophilic blocking reagent HBR used as a control did not show any increase of reaction strength, and its inability to agglutinate human IgM was confirmed. This suggested the possibility that the anti-human IgM monoclonal antibody used in the present invention may be able to suppress those non-specific reactions caused by human IgM that could not be suppressed by conventional reagents for blocking non-specific reactions, by having a clearly different property from the other anti-human IgM monoclonal antibodies that have been used in the conventional reagents for blocking non-specific reactions.

Below, the present invention's effect of suppressing non-specific reactions were verified in the several tests involving immunological reactions (Examples 2 to 4).

### [Example 2]

### Verification of an effect of suppressing non-specific reactions [1] (with the latex reagent for PSA testing)

The present invention's effect on the non-specific reactions seen with the latex reagent for PSA testing, which uses an anti-PSA monoclonal antibody, was verified by measuring the reaction strength in a general automatic analyzer device. PSA is a glycoprotein that has a molecular weight of about 34,000 and is produced specifically in the epithelial cells of the prostate gland, and it is used in the screening (medical examination) for prostate cancer, one of the typical malignant tumors in men of advanced ages.

### (1) Reagents

### (1-1) First reagent

### (i) Basic reagent

30 mM HEPES buffer solution (pH 7.0) containing 0.5 M KCl and 0.1% BSA (bovine serum albumin)

### (ii) The reagent of the present invention

The solution of the anti-human IgM monoclonal antibody t that is capable of inducing immunoagglutination based on an antigen-antibody reaction with human immunoglobulin M by itself was added to the basic reagent above to make the final concentration of the antibody to be 25, 50 or 100 µg/mL.

### (iii) Control reagent

The commercially available heterophilic blocking reagent HBR or a goat anti-human IgM polyclonal antibody (manufactured by the MBC corporation) was added to the basic reagent above to make the final concentration to be 25, 50 or 100 µg/mL.

### (1-2) Second reagent

Nanopia (trademark) PSA, PSA latex reagent Solution 2 (Sekisui Medical corporation)

### (2) Measurement device

Hitachi type 7170 automatic analyzer:

### Parameter conditions

(i) The volumes of the sample, the first reagent, and the second reagent: 4 µL, 100 µL, and 100 µL.
(ii) Method of analysis: 2 point end method (detection points 19-34)
(iii) Measurement wavelengths: main wavelength 570 nm / secondary wavelength 800 nm

### (3) Test samples

### (i) Normal specimen

Serum from a woman was used. Female blood contains little, if any, PSA, and therefore is frequently used as a negative control.

### (ii) Non-specific reaction specimen

Serum from a woman showing an abnormal high-level reaction strength was used. PSA is a male-specific antigen and should not be found in female serum. Thus, a female serum showing an abnormal high-level reaction strength is believed to be inducing a non-specific reaction in which the latex agglutination induced is unrelated to PSA content. Specifically, a female serum showing a high level of absorbance was identified by screening commercially available sera (Serum from Anticoagulant-Free Whole Blood, Normal Female, TENNESSEE BLOOD SERVICES, INC.) with the combined use of the basic reagent and the second reagent mentioned above, and this identified serum was used below.

### (4) Assay method

The normal specimen and the non-specific reaction specimen were assayed in the Hitachi type 7170 automatic analyzer, with the first reagent being the basic reagent, the present invention's reagent or the control reagent, and the second reagent being Nanopia (trademark) PSA PSA latex reagent Solution 2, and the reaction strength was examined.

### (5) Assay results

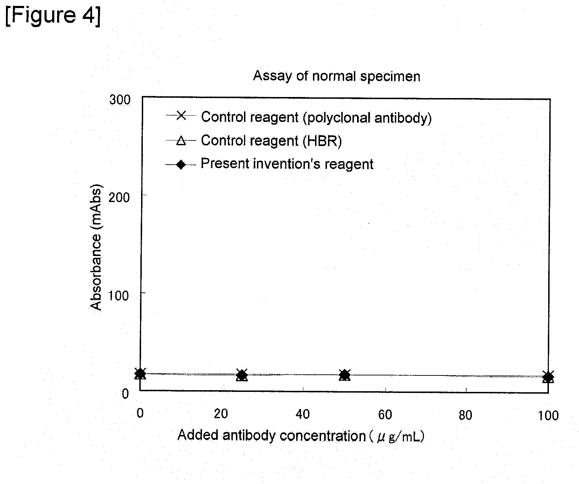

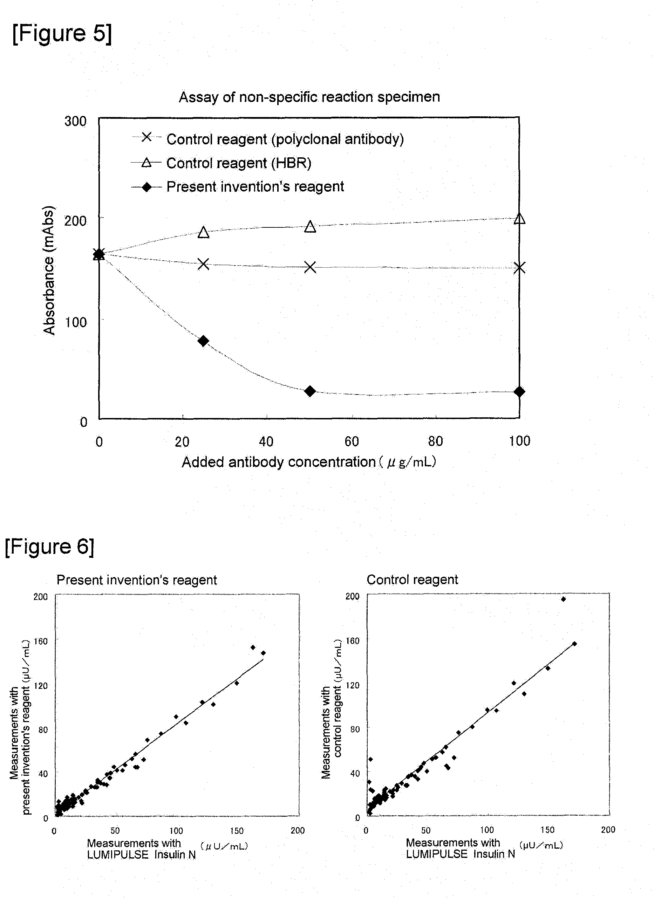

In the assay of the normal specimen shown in Figure 4, either with the present invention's reagent or with the control reagent, an increased concentration of the added anti-human IgM antibody or heterophilic blocking reagent HBR did not result in a change of reaction strength, demonstrating that PSA was absent in the specimen and that an addition of the anti-human IgM antibody or HBR had absolutely no effect on the assay reaction. On the other hand, in the assay of the non-specific reaction specimen shown in Figure 5, an abnormal absorbance of no less than 150 mAbs was observed with the basic reagent having zero additional reagent. The goat anti-human IgM polyclonal antibody, a control reagent, failed to show a significant suppression effect at the added concentrations of up to 100 µg/mL, and the addition of the commercially available heterophilic blocking reagent HBR, which comprises an anti-human IgM monoclonal antibody, even increased the reaction strength, albeit slightly, rather than suppressed it.

When the reagent of the present invention comprising the anti-human IgM monoclonal antibody t was added, at 25 µg/mL, a significant reduction of the abnormal absorbance was recognized, and at 50 µg/mL, the non-specific reaction was almost completely suppressed. It was thus confirmed that the assay method according to the present invention shows a strong effect of suppressing the non-specific reactions caused by human IgM that could not be blocked by the conventional anti-human IgM monoclonal antibodies or anti-human IgM polyclonal antibodies that have been used for the purpose of blocking non-specific reactions.

### [Example 3]

### Verification of an effect of suppressing non-specific reactions [2] (with the latex reagent for CRP testing)

The present invention's effect on the non-specific reactions seen with the latex reagent for CRP testing, which uses an anti-CRP monoclonal antibody, was verified by making measurements in a general automatic analyzer device. CRP is well known as a non-specific inflammation marker.

### (1) Reagent

### (1-1) First reagent

### (i) Basic reagent

20 mM Tris-hydrochloric acid buffer solution (pH 8.5) containing 500 mM sodium chloride.

### (ii) The reagent of the present invention

The solution of the anti-human IgM monoclonal antibody t that is capable of inducing immunoagglutination based on an antigen-antibody reaction with human IgM by itself was added to the basic reagent to make the final concentration of the antibody to be 50 µg/mL.

### (iii) Control reagent

The normal mouse IgG that has a suppression effect on the passive non-specific reactions or the commercially available heterophilic blocking reagent HBR was added to the basic reagent to make the final concentration to be 50 µg/mL.

### (1-2) Second reagent

Type SS Pureauto (trademark) S, CRP latex, latex reagent Solution 2 (Sekisui Medical corporation)

### (1-3) Calibrator

Type SS Pureauto (trademark) S, CRP latex, calibrator (Sekisui Medical corporation)

### (2) Measurement device

Hitachi type 7170 automatic analyzer:

### Parameter conditions

(i) The volumes of the sample, the first reagent, and the second reagent: 3 µL, 150 µL, and 50 µL, respectively.
(ii) Method of analysis: 2 point end method (detection points 19-34)
(iii) Measurement wavelengths: main wavelength 570 nm / secondary wavelength 800 nm
(iv) Calibration: spline

### (3) Test samples

Non-specific reaction specimen 1: an abnormal high-absorbance specimen found among normal sera

Non-specific reaction specimen 2: an abnormal high-absorbance specimen found among normal sera

### (4) Assay method

The non-specific reaction specimens were assayed in the Hitachi type 7170 automatic analyzer device, with the first reagent being the basic reagent, the present invention's reagent or the control reagent and the second reagent being Type SS Pureauto (trademark) S, CRP latex, latex reagent Solution 2, and the measurement values were recorded.

### (5) Assay results

As shown in Table 2, the measurement values for the non-specific reaction specimens 1 and 2 obtained with the basic reagent were 5 to 10 times higher than the normal measurement values (i.e. reference values verified by using a latex reagent from another manufacturer which comprises a rabbit polyclonal antibody. Product name: CRP-Latex (II) "Seiken" X2, Denka Seiken corporation). With the control reagent comprising the normal mouse IgG at 50 µg/mL, improvement of the measurement value was observed for the non-specific reaction specimen 1, but not for the non-specific reaction specimen 2. Thus, it was understood that the non-specific reaction sample 1 was a so-called HAMA specimen comprising heterophile antibodies. With the control reagent comprising the heterophilic blocking reagent HBR at 50 µg/mL, the improvement was observed for both the non-specific reaction specimens 1 and 2, with the measurement values reduced to as low as the normal measurement values. The present invention's reagent comprising the anti-human IgM monoclonal antibody t also improved the measurement values for both the non-specific reaction specimens 1 and 2, and this effect was greater than that shown by the heterophilic blocking reagent HBR. It has been thus confirmed that the assay method of the present invention can suppress the ordinary non-specific reactions that could be strongly inhibited by the heterophilic blocking reagent HBR, with equal or greater effectiveness than the heterophilic blocking reagent HBR.

**[Table 2]**

| | Normal measurement value (Reference value) | Basic reagent | Control reagent | | Present invention's reagent |
|---|---|---|---|---|---|
| | | | Normal mouse IgG | HBR | |
| Non-specific reaction specimen 1 | 1.4 | 15.0 | 1.5 | 1.8 | 1.3 |
| Non-specific reaction specimen 2 | 1.3 | 6.6 | 6.0 | 1.3 | 1.3 |

| | | | | | |
|---|---|---|---|---|---|
| unit (mg/dL) | | | | | |

### [Example 4]

### Verification of an effect of suppressing non-specific reactions [3] (with the latex reagent for insulin testing)

The present invention's effect of suppressing non-specific reactions was also verified with a latex reagent for insulin testing.

### (1) Production of mouse anti-insulin monoclonal antibodies

Two kinds of mouse anti-insulin monoclonal antibodies having different antigen recognition sites were produced through a common method of preparing monoclonal antibodies, with human insulin (30-AI51, manufactured by the Fitzgerald corporation) used as the immunogen. In-house reference numbers 66221 and 66412 were assigned to these antibodies (hereafter referred to as the antibody 66221 and the antibody 66412, respectively).

### (2) Production of latex particles

1,100 g of distilled water, 200 g of styrene, 0.2 g of sodium styrenesulfonate, and a solution in which 1.5 g of potassium persulfate was dissolved in 50 g of distilled water were placed in a glass reaction vial (volume: 2L) equipped with a stirrer device, a chiller for reflux, a temperature detector, a nitrogen inlet tube, and a jacket. The air inside the vial was replaced with nitrogen gas, and polymerization was carried out for 48 hours with continuous stirring at 70°C. After the polymerization was finished, the above solution was subjected to filtration with a filter paper and the latex particles were taken out. Images of the obtained latex particles were taken at 10,000-fold magnification by using a transmission electron microscope device ("type JEM-1010", manufactured by the JEOL corporation), and image analysis was conducted on at least 100 particles to determine the average particle size. The obtained average particle size was 0.3 µm.

### (3) Preparation of solutions of the anti-insulin monoclonal antibody adsorbed latex particles

### 1) Preparation of a solution of the antibody 66221 adsorbed latex particles

To a 1.0% solution of the latex particles having the average particle size of 0.3 µm (5 mM Tris buffer solution, pH 8.5), an equal volume of a solution containing the antibody 66221 at 0.60 mg/mL (5 mM Tris buffer solution, pH 8.5) was added, and the mixture was stirred for 2 hours at 4°C. Subsequently, an equal volume of 5 mM Tris buffer solution (pH 8.5) containing 0.5% BSA was added, and the mixture was stirred for 1 hour at 4°C. The mixture was then centrifuged to remove the supernatant, and the precipitate was re-suspended in 5 mM Tris buffer solution (pH 8.5), to obtain a solution of the antibody 66221 adsorbed latex particles.

### 2) Preparation of a solution of the antibody 66412 adsorbed latex particles

A solution of the antibody 66412 adsorbed latex particles was produced by using the same process described in 1) above with the antibody 66412.

### (4) Reagents

### 1) Preparation of the first reagent

### (i) Basic reagent

5 mM Tris buffer solution (pH 8.5) containing 500 mM sodium chloride and 0.2% BSA.

### (ii) The reagent of the present invention

This reagent was produced by adding a solution of the monoclonal antibody t to the basic reagent to make the final concentration of the antibody to be 50 µg/mL.

### (iii) Control reagent

This reagent was produced by adding the commercially available heterophilic blocking reagent HBR, in stead of the monoclonal antibody t of the present invention, to the final antibody concentration of 50 µg/mL.

### 2) Preparation of the second reagent

Equal volumes of the solution of the antibody 66221 adsorbed latex particles and the solution of the antibody 66412 adsorbed latex particles were mixed, and the mixture was diluted with 5 mM Tris buffer solution (pH 8.5) so that the absorbance at the wavelength of 600 nm was 5.0 Abs. The resultant mixture represented the second reagent.

### (5) Samples

Serum specimens collected for glucose tolerance tests were used as the samples.

### (6) Assay method

(i) Measurements with the present invention's reagent and the control reagent The concentrations of insulin in the sample was measured by using the Hitachi type 7170 automatic analyzer device and combinations of the first and the second reagents. Specifically, 150 µL of the first reagent was added to 10 µL of the sample, the mixture was warmed at 37°C for 5 minutes, and following the subsequent addition of 50 µL of the second reagent, the mixture was stirred. Changes in the absorbance that accompanied the occurrence of agglutination were measured over a period of 5 minutes at the main wavelength of 570 nm and the secondary wavelength of 800 nm, and were compared to a standard curve that had been obtained with standard substances of known concentrations, to compute the insulin concentrations.
(ii) Measurements with the standard reagents

The concentrations of insulin in the sample was measured by using the LUMIPULSE (trademark) Insulin-N (manufactured by the Fujirebio corporation) according to the accompanying manual and the manufacture's recommendations. Figure 6 shows the correlations between this set of insulin measurement values and those obtained above with the present invention's reagent or the control reagent.

### (7) Assay results and discussion

In Figure 6 showing the correlations, some samples failing to exhibit a correlation are recognized in the test with the control reagent (HBR). On the other hand, the lack of correlation is suppressed in the present Example in which the present invention's reagent has been used, where excellent correlations with the LUMIPULSE (trademark) insulin-N are apparent. It has been thus confirmed that, also in the insulin testing, the reagent of the present invention suppresses non-specific reactions that could not be suppressed by the commercially available heterophilic blocking reagent HBR.

### Industrial Applicability

Since the monoclonal antibody used in the present invention is selected on the basis of the potency to induce agglutination reactions in solution, it reacts efficiently with human IgM, enabling a more accurate and consistent immunoagglutination assay for human IgM.

The monoclonal antibody used in the present invention also provides a high-quality and inexpensive assay reagent and assay kit for immunoagglutination assay for human

The present disclosure's agent for suppressing non-specific reactions, as well as the immunoassay using the said agent, can suppress ordinary non-specific reactions caused by HAMA or the like. In addition, they can also suppress even those non-specific reactions that could not be prevented by the known measures, irrespective of the type of the test item, provided that these non-specific reactions are possibly caused by human IgM, thus enabling more accurate measurements than the conventional assays.

### [Reference to the deposited microorganism]

A. Name and address of the depositary institution to which the biological material was deposited:
   International Patent Organism Depositary,
   The National Institute of Advanced Industrial Science and Technology
      Chuo 6, Higashi 1-1-1, Tsukuba-City, Ibaraki, 305-8566 JAPAN
B. Date on which the biological material was deposited to the above depositary institution:
   September 19, 2008 (the date of the original deposition)
   June 9, 2009 (the date on which the original deposition was changed to a deposition according to the Budapest Treaty)
C. Deposition number assigned to the deposition by the above depositary institution:

## Claims

1. An immunoassay for determining the quantity of an analyte in a sample based on an antigen-antibody reaction between the analyte and an assay antibody or an assay antigen,
wherein non-specific reactions are suppressed by the addition of an anti-human IgM monoclonal antibody that reacts specifically with human IgM and is capable of inducing immunoagglutination by itself based on an antigen-antibody reaction with human IgM in solution, or a functional fragment derived from said anti-human IgM antibody,
wherein the functional fragment comprises a Fab region derived from the said anti-human IgM antibody,
and wherein the analyte is not human IgM.

2. The immunoassay according to claim 1, wherein the anti-human IgM monoclonal antibody is selected from (a) or (b) below:
(a) A monoclonal antibody produced by FERM BP-11134 hybridoma;
(b) An antibody that shows a cross-reactivity with the antibody according to (a) above.

3. The immunoassay according to any of claims 1 to 2, wherein the assay antibody or the assay antigen is supported on an insoluble carrier.

4. The immunoassay according to claim 3, wherein the insoluble carrier comprises latex, colloidal metal, or silica.

5. The immunoassay according to any of claims 1 to 4, wherein the anti-human IgM monoclonal antibody is put into contact with the sample before a step of bringing the assay antibody or the antigen into contact with the sample.

6. Use of an anti-human IgM monoclonal antibody that reacts specifically with human IgM and is capable of inducing immunoagglutination by itself based on an antigen-antibody reaction with human IgM in solution, or a functional fragment derived from said anti-human IgM antibody for suppressing non-specific reactions in an immunoassay for determining the quantity of an analyte in a sample based on an antigen-antibody reaction between the analyte and an assay antibody or an assay antigen,
wherein the functional fragment comprises a Fab region derived from the said anti-human IgM antibody,
and wherein the analyte is not human IgM.

7. The use according to claim 6, wherein the anti-human IgM monoclonal antibody is put into contact with the sample before a step of bringing the assay antibody or the antigen into contact with the sample.

## Patentansprüche

1. Immunassay zum Bestimmen der Menge eines Analyts in einer Probe basierend auf einer Antigen-Antikörper-Reaktion zwischen dem Analyten und einem Assay-Antikörper oder einem Assay-Antigen,
wobei nicht-spezifische Reaktionen durch die Zugabe eines monoklonalen Antikörpers gegen humanes IgM, welcher spezifisch mit humanem IgM reagiert und fähig ist, basierend auf einer Antigen-Antikörper-Reaktion mit humanem IgM in Lösung alleine Immunagglutination zu induzieren, oder eines von diesem Antikörper gegen humanes IgM abgeleiteten funktionalen Fragments unterdrückt werden,
wobei das funktionale Fragment eine Fab-Region, die von diesem Antikörper gegen humanes IgM abgeleitet ist, umfasst,
und wobei der Analyt nicht humanes IgM ist.

2. Immunassay gemäß Anspruch 1, wobei der monoklonale Antikörper gegen humanes IgM ausgewählt ist aus untenstehendem (a) oder (b):
(a) Ein durch FERM BP-11134-Hybridom hergestellter monoklonaler Antikörper;
(b) Ein Antikörper, der eine Kreuzreaktivität mit dem obigen Antikörper gemäß (a) aufweist.

3. Immunassay gemäß einem der Ansprüche 1 oder 2, wobei der Assay-Antikörper oder das Assay-Antigen auf einem unlöslichen Träger getragen wird.

4. Immunassay gemäß Anspruch 3, wobei der unlösliche Träger Latex, kolloidales Metall oder Silika umfasst.

5. Immunassay gemäß einem der Ansprüche 1 bis 4, wobei der monoklonale Antikörper gegen humanes IgM vor einem Schritt des Inkontaktbringens des Assay-Antikörpers oder des Antigens mit der Probe mit der Probe in Kontakt gebracht wird.

6. Verwendung eines monoklonalen Antikörpers gegen humanes IgM, welcher spezifisch mit humanem IgM reagiert und fähig ist, basierend auf einer Antigen-Antikörper-Reaktion mit humanem IgM in Lösung alleine Immunagglutination zu induzieren, oder eines von diesem Antikörper gegen humanes IgM abgeleiteten funktionalen Fragments, zum Unterdrücken nicht-spezifischer Reaktionen in einem Immunassay zum Bestimmen der Menge eines Analyts in einer Probe basierend auf einer Antigen-Antikörper-Reaktion zwischen dem Analyten und einem Assay-Antikörper oder einem Assay-Antigen,
wobei das funktionale Fragment eine Fab-Region, die von diesem Antikörper gegen humanes IgM abgeleitet ist, umfasst,
und wobei der Analyt nicht humanes IgM ist.

7. Verwendung gemäß Anspruch 6, wobei der monoklonale Antikörper gegen humanes IgM vor einem Schritt des Inkontaktbringens des Assay-Antikörpers oder des Antigens mit der Probe mit der Probe in Kontakt gebracht wird.

## Revendications

1. Dosage immunologique pour déterminer la quantité d'un analyte dans un échantillon sur la base d'une réaction antigène-anticorps entre l'analyte et un anticorps de test ou un antigène de test,
dans lequel des réactions non spécifiques sont supprimées par l'ajout d'un anticorps monoclonal anti-IgM humaine qui réagit spécifiquement avec l'IgM humaine et est capable par lui-même d'induire une immunoagglutination sur la base d'une réaction antigène-anticorps avec une IgM humaine en solution, ou un fragment fonctionnel dérivé dudit anticorps anti-IgM humaine,
dans lequel le fragment fonctionnel comprend une région Fab dérivée dudit anticorps anti-IgM humaine,
et dans lequel l'analyte n'est pas une IgM humaine.

2. Dosage immunologique selon la revendication 1, dans lequel l'anticorps monoclonal anti-IgM humaine est sélectionné parmi (a) ou (b) ci-dessous :
(a) un anticorps monoclonal produit par l'hybridome FERM BP-11134 ;
(b) un anticorps qui présente une réactivité croisée avec l'anticorps selon le point (a) ci-dessus.

3. Dosage immunologique selon l'une quelconque des revendications 1 à 2, dans lequel l'anticorps de test ou l'antigène de test est supporté sur un support insoluble.

4. Dosage immunologique selon la revendication 3, dans lequel le support insoluble comprend du latex, un métal colloïdal ou de la silice.

5. Dosage immunologique selon l'une quelconque des revendications 1 à 4, dans lequel l'anticorps monoclonal anti-IgM humaine est mis en contact avec l'échantillon avant une étape de mise en contact de l'anticorps ou de l'antigène de test avec l'échantillon.

6. Utilisation d'un anticorps monoclonal anti-IgM humaine qui réagit spécifiquement avec l'IgM humaine et est capable par lui-même d'induire une immunoagglutination sur la base d'une réaction antigène-anticorps avec une IgM humaine en solution, ou un fragment fonctionnel dérivé dudit anticorps anti-IgM humaine pour supprimer des réactions non spécifiques dans un dosage immunologique pour déterminer la quantité d'un analyte dans un échantillon sur la base d'une réaction antigène-anticorps entre l'analyte et un anticorps de test ou un antigène de test,
dans lequel le fragment fonctionnel comprend une région Fab dérivée dudit anticorps anti-IgM humaine,
et dans lequel l'analyte n'est pas une IgM humaine.

7. Utilisation selon la revendication 6, dans laquelle l'anticorps monoclonal anti-IgM humaine est mis en contact avec l'échantillon avant une étape de mise en contact de l'anticorps ou de l'antigène de test avec l'échantillon.
